# FASCICULE DE BREVET EUROPEEN

(11) **EP 2 413 705 B1**
(45) Date de publication et mention de la délivrance du brevet: **16.01.2013**
(21) Numéro de dépôt: 10703188.2
(22) Date de dépôt: 09.02.2010
(51) Int. Cl.: A23C 9/142, A23C 9/152, A23L 1/29, A61K 8/98, A61K 35/20, A61Q 19/08

(54) **PROCEDE DE FABRICATION D'UN PRODUIT LAITIER A TENEUR REDUITE EN GLUCIDES ET ENRICHI EN PHOSPOLIPIDES, NOTAMMENT EN CHOLINE, PROTEINES ET CALCIUM**
VERFAHREN ZUR HERSTELLUNG EINES KOHLENHYDRATREDUZIERTEN MILCHPRODUKTS, DAS MIT PHOSPHOLIPIDEN, INSBESONDERE CHOLIN, PROTEINEN UND CALCIUM ANGEREICHERT IST
METHOD FOR MANUFACTURING A DAIRY PRODUCT HAVING A REDUCED CARBOHYDRATE CONTENT AND ENRICHED WITH PHOSPHOLIPIDS, IN PARTICULAR CHOLINE, PROTEINS, AND CALCIUM

(30) Priorité: 02.04.2009 FR 0952160
(43) Date de publication de la demande: 08.02.2012
(73) Titulaire: Sojasun Technologies, 35330 Noyal-sur-vilaine (FR)
(72) Inventeur: EFSTATHIOU, Théo, F-35740 Pace (FR)
(74) Mandataire: Larcher, Dominique
(86) Numéro de dépôt international: PCT/EP2010/051569
(87) Numéro de publication internationale: WO 2010/112255

(56) Documents cités:
- EP-A- 0 491 623
- WO-A-2006/128465
- GB-A- 1 362 502
- US-A- 5 284 941
- SACHDEVA S ET AL: "RECOVERY OF PHOSPHOLIPIDS FROM BUTTERMILK USING MEMBRANE PROCESSING" KIELER WIRTSCHAFTLICHE FORSCHUNGSBERICHTE, VERLAG TH. MANN, GELSENKIRCHEN, DE, vol. 49, no. 1, 1 janvier 1997 (1997-01-01), pages 47-68, XP001014232 ISSN: 0023-1347
- ROMBAUT R ET AL: "Microfiltration of Butter Serum Upon Casein Micelle Destabilization" JOURNAL OF DAIRY SCIENCE, AMERICAN DAIRY SCIENCE ASSOCIATION, SAVOY, IL, US, vol. 89, no. 6, 1 juin 2006 (2006-06-01), pages 1915-1925, XP002535317 ISSN: 0022-0302

## Description

Le domaine de l'invention est celui des produits laitiers. En particulier, la présente invention se rapporte à un procédé de fabrication d'un produit laitier à teneur réduite en glucides et enrichi en phospholipides, notamment en choline, protéines et calcium, à partir du babeurre.

Les connaissances dans le domaine de la nutrition aussi bien relatives aux apports et besoins nutritionnels qu'aux conséquences de l'alimentation sur la santé humaine se sont considérablement développées ces dernières années. Il est désormais admis que l'alimentation doit non seulement fournir les nutriments nécessaires à la couverture des besoins métaboliques en quantités adéquates, mais que ceux-ci doivent aussi contribuer à l'amélioration de la santé (Weber , 2001, Role of biomarkers in nutritional science and industry, Brit., J. Nutri., 86, 51, 593-595; Chanussot F., 2008, lécithine, métabolisme et nutrition, éditions TEC & DOC, Lavoisier).

Afin de réduire les facteurs de risques de maladie, prévenir le vieillissement, maintenir un état de santé acceptable tout en satisfaisant les besoins métaboliques, il est donc conseillé d'adopter une alimentation se rapprochant des apports préconisés par les experts nutritionnistes nationaux et internationaux. Ainsi, il est recommandé de limiter les apports en glucides et en lipides totaux, tout en favorisant des apports adéquats en protéines et en phospholipides, notamment en choline.

En effet, la choline est considérée comme un nutriment essentiel présent dans divers aliments sous forme libre ou incluse dans des phospholipides tels que la phosphatidylcholine, la sphingomyléine, la phosphocholine ou encore la glycerophosphocholine. De nombreuses études scientifiques ont montré l'intérêt nutritionnel de la choline. Ainsi, la choline a été décrite comme nécessaire au fonctionnement normal de l'ensemble des cellules, quel que soit l'âge ou le sexe des individus, y compris la fonction nerveuse et cérébrale, le métabolisme hépatique et le transport des nutriments à travers le corps (Steven H. Zeisel, 2007, Nutr Today). En outre, des études ont mis en évidence que la choline est associée à la réduction du risque du cancer du sein (Xiran xu et al., 2008, The FASEB Journal) et également à la réduction du risque de cardiopathies (Bidulescu A et al., 2007, BMC Cardiovasc Disord.). En effet, la choline, précurseur de la bétaïne est impliquée dans la destruction de l'homocystéine, un acide aminé présent dans le sang qui serait associé au risque accru de cardiopathies. De plus, les recherches indiquent que la choline est essentielle au bon développement cérébral des foetus et des nourrissons et permet de prévenir les défectuosités de naissance (Steven H. Zeisel, 2007, Nutr Today). Au regard de ces études, l'Institut de Médecine de l'Académie des Sciences Américaine (*« The Institute of Medicine of the US National Academy of Sciences »*) préconise l'apport de choline notamment pour la santé du rein, du foie, contre la dépression et la maladie de Parkison mais aussi en vue de l'atténuation des odeurs corporelles (personne souffrant de trimethylaminuria). En outre, cet Institut recommande, depuis 1998, un apport journalier de choline de 550 mg pour un homme adulte et de 425 mg pour une femme adulte (Zeisel, 2000, J Am Coll Nutr.). Ces valeurs correspondent à la somme de phosphatidylcholine, la sphingomyléine, la phosphocholine et de la glycerophosphocholine, exprimée en équivalent choline à laquelle est ajoutée la choline libre.

Or, à l'heure actuelle, les études réalisées par les experts nutritionnistes indiquent une sous-consommation en phospholipides, notamment en choline, avec un déficit quotidien de l'ordre de 30 à 50 % (Canty et Zeisel, 1994, Nutr Rev.). Ainsi, seul 10 % des américains consomment actuellement la quantité recommandée en choline. Il est donc utile de rétablir une consommation adéquate par de nouveaux aliments ou ingrédients alimentaires à teneur réduite en glucides et enrichis en protéines et en phospholipides, notamment en choline.

Il existe à l'heure actuelle des ingrédients, notamment laitiers, enrichis en phospholipides. Toutefois, ces ingrédients peuvent être dépourvus de protéines, présenter une teneur relativement faible en phospholipides, en protéines et/ou en calcium, présenter une teneur élevée en composés indésirables, avoir un coût de revient élevé, une texture et/ou un goût inacceptable.

Ainsi, par exemple, un oeuf pesant 60 grammes contient 125,5 mg de choline, soit environ un quart de la quantité quotidienne recommandée, mais présente un inconvénient majeur puisqu'il apporte également 300 mg de cholestérol.

Sachdeva et al., Kieler wirtschaftliche Forschungsberichte, vol. 49(1), pages 47-68 divulgue un procédé pour obtenir des phospholipides à partir de babeurre.

Les inventeurs ont maintenant mis au point un procédé qui permet de résoudre en tout ou partie les problèmes évoqués ci-dessus, ledit procédé permettant la fabrication d'un produit laitier à teneur réduite en glucides et enrichi en phospholipides, notamment en choline, protéines et calcium à partir du babeurre.

Ainsi selon un premier aspect, l'invention a pour objet un procédé de préparation d'un produit laitier, caractérisé en ce qu'il comprend les étapes suivantes :
a) traitement thermique d'un babeurre à une température comprise entre 50 et 90 °C, de préférence entre 60 et 80 °C et tout préférentiellement à 80 °C, pendant une durée comprise entre 30 secondes et 60 minutes de préférence entre 1 et 30 minutes, et tout préférentiellement 1 minute ;
b) homogénéisation dudit babeurre traité thermiquement obtenu à l'étape a)
c) ultrafiltration du babeurre traité thermiquement et homogénéisé obtenu à l'étape b) à un pH compris dans une gamme allant de 4,5 à 5,8 ou de 5,5 à 6, de préférence à pH 4,7 ou pH 5,8 et tout préférentiellement à pH 5,8, sur des membranes ayant un seuil de coupure compris entre 1 000 et 20 000 Da, de préférence entre 5 000 et 15 000 Da, et tout préférentiellement de 10 000 Da.

Le procédé de fabrication d'un produit laitier selon l'invention présente notamment les avantages suivants :
- Il est simple, rapide, adapté à l'échelle industrielle et économique du fait qu'il utilise un coproduit de fabrication du beurre, le babeurre. Le procédé selon l'invention est en effet particulièrement intéressant puisqu'il permet de valoriser un coproduit du beurre et permet de faire face à la raréfaction et l'augmentation du prix des matières premières.
- Il permet d'obtenir un produit laitier à teneur réduite en cholestérol, en glucides et enrichi en protéines, calcium et phospholipides, notamment en sphingomyléine et en choline. En effet, le procédé selon l'invention permet de réduire de moitié la teneur en glucides du babeurre et ainsi d'obtenir un produit présentant environ 10 % de glucides lorsque le babeurre est issu du lait de vache. Ceci est particulièrement intéressant d'une part pour se rapprocher des apports en glucides préconisés par les nutritionnistes et d'autre part pour limiter l'apport en lactose, glucide majoritaire du lait, qui est allergène et mal toléré par certaines personnes déficientes en lactase. En outre, le procédé selon l'invention permet de s'absoudre de la mise en oeuvre d'étape spécifique pour l'élimination des glucides, telle que l'utilisation de lactases, qui peut être longue et coûteuse. Parallèlement, le procédé selon l'invention permet de concentrer jusqu'à environ 20 fois la quantité de protéines présentes dans le babeurre, jusqu'à environ 10 fois la quantité de calcium présent dans le babeurre, jusqu'à environ 25 fois la quantité de phospholipides totaux dont jusqu'à environ 10 fois la quantité de choline totale et jusqu'à environ 70 fois la quantité de sphingomyéline présents dans le babeurre. Les inventeurs ont ainsi mis au point un procédé qui permet de concentrer efficacement les protéines, phospholipides et le calcium en utilisant une étape d'ultrafiltration tout en évitant d'une part le colmatage des membranes dû notamment aux protéines, d'autre part en évitant le passage des phospholipides et du calcium dans le filtrat et enfin en évitant l'utilisation de solvants organiques, interdits en agro-alimentaire.

- Il permet d'obtenir un produit laitier possédant des propriétés physiques, fonctionnelles et des qualités organoleptiques satisfaisantes. En effet, le produit laitier obtenu présente les caractéristiques d'une crème laitière, onctueuse et lisse en bouche.
- Il permet d'obtenir un produit laitier facilement utilisable tel quel ou sous forme de poudre comme ingrédient alimentaire.
- Il permet d'obtenir un produit laitier relativement stable dans le temps.
- Il permet d'obtenir un produit laitier présentant les caractéristiques d'un agent de texture, en particulier d'un agent anti-synérèse, agent lissant, agent émulsifiant, agent d'onctuosité, agent de texture crémeuse.

Afin de permettre une meilleure compréhension de la présente invention, certaines définitions sont fournies. Sauf indications particulières, les autres termes techniques employés dans la présente Demande doivent être interprétés selon leur sens habituel.

On entend par « choline » au sens de la présente invention, la somme de phosphatidylcholine, la sphingomyléine, la phosphocholine et de la glycerophosphocholine, exprimée en équivalent choline à laquelle est ajoutée la choline libre.

On entend par « produit laitier » au sens de la présente invention tout produit issu du lait.

On entend par « babeurre », au sens de la présente invention un coproduit de la fabrication du beurre obtenu lors du processus d'inversion de phase en continu ou en discontinu, appelé barattage, de la crème de lait ou de la crème de lactosérum, de préférence de la crème de lait.

Le babeurre peut être obtenu à partir du lait de tout animal tel que le lait de vache, de chèvre, de brebis, de bufflesse, de préférence à partir du lait de vache.

En particulier, le babeurre utilisé à l'étape a) est un babeurre acide, à savoir dont le pH est compris dans une gamme allant de 4,5 à 5,8 ou de 5,5 à 6, de préférence à pH 4,7 ou pH 5,8 et tout préférentiellement à pH 5,8. Le babeurre acide peut être obtenu lors de la transformation de crème acidifiée en beurre. Il peut également être obtenu par acidification à partir de babeurre doux. L'acidification du babeurre peut être obtenue par toutes techniques bien connues de l'Homme du Métier telles que par l'utilisation d'acide chlorhydrique ou avantageusement par l'utilisation de la glucono-delta-lactone (GDL) qui implique une acidification lente. Ce mode d'acidification par la glucono-delta-lactone est cinétiquement le plus proche de celui que les bactéries lactiques réalisent. En outre, l'utilisation de ce composé est autorisée en industrie fromagère, pour les ajustements de pH.

En particulier, lorsque le babeurre utilisé à l'étape a) est un babeurre acide, à savoir dont le pH est compris dans une gamme allant de 4,5 à 5,8, en particulier à pH 4,7, le produit laitier obtenu selon le procédé selon l'invention est gélifié, ferme et lisse. Un tel produit est particulièrement utile pour la préparation de compositions alimentaires telles que les boissons, les desserts lactés et les sauces.

En particulier, lorsque le babeurre utilisé à l'étape a) est un babeurre acide, à savoir dont le pH est compris dans une gamme allant de 5,5 à 6 en particulier à pH 5,8, le produit laitier obtenu selon le procédé selon l'invention présente un aspect de crème visqueuse, et est onctueux, lisse avec un goût de crème. Un tel produit est particulièrement utile pour la préparation de compositions alimentaires telles que les boissons et les desserts lactés.

Selon un mode de réalisation particulier du procédé de fabrication d'un produit laitier selon l'invention, l'étape a) de traitement thermique peut être mise en oeuvre à une température de 60 °C pendant 30 minutes ou à une température de 80 °C pendant 1 minute. À l'échelle industrielle, l'étape a) peut être avantageusement mise en oeuvre à une température comprise entre 80°C et 90°C pendant 30 secondes à 2 minutes, préférentiellement 1 minute.

L'étape a) de traitement thermique mise en oeuvre à une température comprise entre 60 et 80 °C, tout préférentiellement à 80°C pendant une durée comprise entre 1 et 30 minutes, tout préférentiellement pendant une minute présente l'avantage de pasteuriser le babeurre tout en optimisant l'obtention d'un produit présentant des propriétés physiques, fonctionnelles et des qualités organoleptiques satisfaisantes. En effet, le produit laitier obtenu présente les caractéristiques d'une crème laitière, onctueuse et lisse en bouche.

Selon un mode de réalisation particulier, l'étape b) d'homogénéisation, est mise en oeuvre à une température comprise entre 40 et 60 °C, de préférence entre 45 et 55 °C, et tout préférentiellement à 50 °C.

Selon un mode de réalisation particulier, l'étape b) d'homogénéisation, est mise en oeuvre à une température comprise entre 40 et 60 °C, de préférence entre 45 et 55 °C, et tout préférentiellement à 50 °C et à une pression comprise entre 160 et 280 bars, de préférence entre 180 et 240 bars, et tout préférentiellement à 220 bars.

L'étape b) d'homogénéisation mise en oeuvre à une température comprise entre 45 et 55 °C, et tout préférentiellement à 50 °C et à une pression comprise entre 180 et 240 bars, et tout préférentiellement à 220 bars, présente l'avantage d'optimiser l'obtention d'un produit lisse et stable.

L'étape c) d'ultrafiltration à un pH compris dans une gamme allant de 4,5 à 5,8 ou de 5,5 à 6, de préférence à pH 4,7 ou pH 5,8 et tout préférentiellement à pH 5,8 présente l'avantage d'optimiser la prévention du colmatage des membranes. Ces gammes de pH et pH particuliers peuvent être obtenus par toutes techniques bien connues de l'Homme du Métier telles que par l'utilisation d'acide chlorhydrique ou avantageusement par l'utilisation de la glucono-delta-lactone (GDL).

L'étape c) d'ultrafiltration sur des membranes ayant un seuil de coupure compris entre 5 000 et 15 000 Da, et tout préférentiellement de 10 000 Da, permet d'optimiser la concentration en protéines, calcium et phospholipides tout en optimisant la réduction de glucides.

L'étape c) d'ultrafiltration peut être mise en oeuvre sur des membranes organiques telles que des membranes organiques spiralées ou des membranes inorganiques telles que des membranes en céramique.

Selon un mode de mise en oeuvre particulier du procédé selon l'invention, l'étape c) d'ultrafiltration est mise en oeuvre sur des membranes organiques, en particulier sur des membranes organiques spiralées.

L'étape c) d'ultrafiltration sur des membranes organiques présente l'avantage d'avoir un coût peu élevé.

L'étape c) d'ultrafiltration sur des membranes organiques spiralées présente l'avantage d'avoir un ratio « surface membrane/volume d'encombrement » important et de permettre le travail à hautes pressions.

Selon un mode de mise en oeuvre particulier du procédé selon l'invention, l'étape c) d'ultrafiltration est mise en oeuvre à une pression comprise entre 0 et 15 bars, de préférence entre 0 et 10 bars. En particulier, la pression à l'entrée de la membrane d'ultrafiltration peut être comprise entre 2 et 10 bars et la pression à la sortie de la membrane d'ultrafiltration peut être comprise entre 0 et 5 bars.

L'étape c) d'ultrafiltration mise en oeuvre à une pression comprise entre 0 et 15 bars présente l'avantage d'éviter le colmatage des membranes tout en concentrant au maximum les composés d'intérêts, notamment les phospholipides, protéines et calcium..

Lors de l'étape c), le babeurre traité thermiquement et homogénéisé obtenu à l'étape b) et/ou le retentât peuvent être maintenu à une température comprise entre 40 et 60 °C, de préférence entre 45 et 55 °C et tout préférentiellement à 50°C.

L'étape c) d'ultrafiltration au cours de laquelle le babeurre traité thermiquement et homogénéisé obtenu à l'étape b) et/ou le retentât est maintenu à une température comprise entre 40 et 60 °C, présente l'avantage de maintenir, la forme lisse, visqueuse et suffisamment fluide du retentât obtenu, et de rendre les conditions de déshydratation (étape d) et/ou de conditionnement (étape e) optimales.

Selon un mode de mise en oeuvre particulier du procédé selon l'invention, au moins une des étapes a) et b) est mise en oeuvre à un pH compris dans une gamme allant de 4,5 à 5,8 ou de 5,5 à 6, de préférence à pH 4,7 ou pH 5,8 et tout préférentiellement à pH 5,8. La mise en oeuvre de ces étapes à ces pH particuliers présente l'avantage d'éviter le colmatage des membranes lors de l'étape c) d'ultrafiltration.

Le procédé selon l'invention permet d'obtenir un produit après l'étape c) dont la quantité de protéines a été concentrée environ 5 fois par rapport à celle présente dans le babeurre, dont la quantité de calcium a été concentrée environ 2,5 fois par rapport à celle présente dans le babeurre et dont la quantité de phospholipides totaux a été concentrée environ 6 fois, dont 2 fois pour la quantité de choline et 15 fois pour la quantité de sphingomyéline, par rapport à celle présente dans le babeurre.

Le procédé selon l'invention peut comprendre en outre l'étape suivante :
d) déshydratation du retentât obtenu à l'étape c), de préférence par atomisation.

La mise en oeuvre de l'étape d) de déshydratation présente notamment les avantages suivants :
- d'optimiser l'enrichissement du produit laitier en phospholipides, protéines et calcium en concentrant les composants du retentât obtenu à l'étape c). En effet, le procédé selon l'invention incluant l'étape d) de déshydratation permet de concentrer environ 20 fois la quantité de protéines présentes dans le babeurre, environ 10 fois la quantité de calcium présent dans le babeurre, environ 25 fois la quantité de phospholipides totaux dont 10 fois la quantité de choline et 70 fois la quantité de sphingomyéline présents dans le babeurre; et
- d'améliorer la stabilité et le stockage du produit laitier ainsi obtenu.

La déshydratation du retentât peut se faire par toute technique bien connue de l'Homme du Métier telles que par atomisation, lyophilisation, séchage à rouleaux.

L'étape de déshydratation par atomisation présente l'avantage d'optimiser la solubilité du produit laitier ainsi obtenu.

Le procédé selon l'invention peut comprendre en outre l'étape suivante :
e) conditionnement du retentât obtenu à l'étape c) ou du retentât déshydraté obtenu à l'étape d).

En particulier, l'étape e) de conditionnement est mise en oeuvre à bref délai et de préférence immédiatement après l'étape c) ou d) afin d'éviter l'oxydation du produit. Le produit laitier ainsi obtenu est de préférence stocké à une température comprise dans une gamme allant de 4 à 20°C.

Selon un second aspect, l'invention a également pour objet un produit laitier susceptible d'être obtenu par un procédé selon l'invention. Le produit laitier selon l'invention présente notamment l'avantage d'avoir une teneur réduite en glucides et d'être enrichi en protéines, calcium et phospholipides, notamment en sphingomyléine et en choline et d'avoir un faible coût de production.

L'invention a également pour objet une composition comprenant un produit laitier selon l'invention, ladite composition étant choisie dans le groupe comprenant les compositions alimentaires, les compositions pharmaceutiques, et les compositions cosmétiques.

On entend par « composition alimentaire » au sens de l'invention, tout type de composition apte à l'alimentation humaine ou animale. Les compositions alimentaires comprennent les compositions diététiques (tels que des Aliments Diététiques Destinés à des Fins Médicales Spéciales ; exemple des produits pour seniors fragiles), les alicaments ou « aliments santé » et compléments alimentaires (compositions pour satisfaire les besoins métaboliques, maintenir la santé, prévenir les pathologies et le vieillissement).

Avantageusement, les compositions alimentaires selon l'invention peuvent être des compositions répondant simultanément aux critères suivants :
- Naturalité : compositions issues de l'agriculture biologique ;
- Plaisir : compositions avec des qualités organoleptiques très appréciées : compositions onctueuses, lisses en bouche ;
- Santé : compositions nutritionnellement équilibrées (à teneur réduite en glucides et lipides totaux , notamment en cholestérol), compositions pour satisfaire les besoins métaboliques, compositions pour maintenir la santé, compositions pour prévenir les pathologies et le vieillissement.

Les compositions alimentaires selon l'invention peuvent notamment prendre toute forme d'aliment et en particulier peuvent être choisis parmi le lait, les produits à base de laits fermentés tels que les yogourts, les yaourts, les fromages frais, les desserts lactés, les spécialités fromagères à tartiner, les boissons, les purées, les potages, les crèmes glacées, les formules infantiles, les aliments pour animaux de compagnie, les produits à base de céréales, en particulier à base de soja.

Les compositions alimentaires selon l'invention peuvent comprendre entre 0,1 et 99 % de produit laitier selon l'invention, selon l'effet recherché.

Les compositions alimentaires selon l'invention peuvent comprendre en outre d'autres ingrédients bioactifs tels que des antioxydants (vitamines, polyphénols, coenzyme Q10), des prébiotiques, des probiotiques, des acides gras oméga 3, du collagène ou des hydrolysats de collagène.

Les produits laitiers selon l'invention peuvent être inclus dans des compositions afin d'obtenir un apport en protéines, calcium et/ou en phospholipides, en particulier en choline et/ou sphingomyéline. Les compositions selon l'invention comprenant un produit laitier selon l'invention peuvent également être utiles afin d'obtenir un apport en protéines, calcium et/ou phospholipides, en particulier en choline et/ou sphingomyéline, chez un sujet humain ou animal.

La présente invention a ainsi également pour objet l'utilisation non thérapeutique d'un produit laitier selon l'invention ou d'une composition selon l'invention, ladite composition étant une composition alimentaire, en vue d'un apport en vue d'un apport en protéines, calcium et/ou phospholipides, en particulier en choline et/ou sphingomyéline. Le produit selon l'invention présente l'avantage d'être compatible avec un régime diététique et de palier à d'éventuelles carences d'un tel régime, notamment en calcium, phospholipides et protéines.

Comme indiqué précédemment, les propriétés nutritionnelles du produit laitier selon l'invention se définissent notamment au niveau des phospholipides, des protéines, et du calcium :
- De nombreuses études scientifiques ont montré l'intérêt nutritionnel des phospholipides (Dewettinck et al., 2008, International Dairy Journal, 18, 436-457). Ainsi, les phospholipides possèdent des propriétés anti-inflammatoires, anti-athérogènes, ils jouent un rôle, notamment la sphingomyéline, dans la prévention des maladies cardio-vasculaires telles que l'atherosclérose (Nofer et al., 2008, circulation journal of the american heart association ; Nilsson et al., 2006, Journal of Lipid Research). Ainsi, la choline a été décrite comme nécessaire au fonctionnement normal de l'ensemble des cellules, quel que soit l'âge ou le sexe des individus, y compris la fonction nerveuse et cérébrale, le métabolisme hépatique et le transport des nutriments à travers le corps (Steven H. Zeisel, 2007, Nutr Today). En outre, des études ont mis en évidence que la choline est associée à la réduction du risque du cancer du sein (Xiran xu et al., 2008, The FASEB Journal) et également à la réduction du risque de cardiopathies (Bidulescu A et al., 2007, BMC Cardiovasc Disord.). Les études ont également montré qu'un apport en sphingolipides (phospholipides particuliers) permet d'inhiber la carcinogenèse, en particulier dans le cas du cancer du côlon (Dewettinck et al., 2008, International Dairy Journal, 18, 436-457) ; ils jouent également un rôle hypocholestérolémiant et anti-infectieux (Vesper et al.,1999, Amercican Society for Nutritional Sciences). En particulier, la phosphatidylcholine et la phosphatidylsérine sont nécessaires pour la structure et le fonctionnement normal des cellules (Hannahan et Nelson, 1984, J. Lipid Res.). Une complémentation en phosphatidylcholine aide à baisser le taux de cholesterol (Wojciki et al., 1995, Phytoter. Res), aide à la détoxification du foie (Knüchel, 1979, Med. Welt.) et au bon fonctionnement du cerveau (Sitaram et al., 1978, Science). Des études ont montré que l'administration de phosphatidylsérine permet d'améliorer des facultés cognitives associées au vieillissement (Delwaide et al., 1986, Acta. Neurol. Scand.) Dans divers essais, les chercheurs ont observé le rôle bénéfique des phospholipides sur les troubles liés à la dépression chez les personnes âgées (Maggioni et al., 1990, Acta Psychiatr Scand). Les recherches indiquent que la choline est essentielle au bon développement cérébral des foetus et des nourrissons et permet de prévenir les défectuosités de naissance (Steven H. Zeisel, 2007, Nutr Today). De plus, l'Institut de Médecine de l'Académie des Sciences Américaine préconise l'apport de choline notamment contre la dépression et la maladie de Parkinson. Une complémentation en phosphatidylcholine permet d'améliorer la résistance à l'effort physique, notamment chez les sportifs de haut niveau (Von Allwôrden et al., 1993, Eur. J Appl Physiol.).

Des études ont également montré l'intérêt des phospholipides dans la fabrication de fromages. Ainsi, il a été démontré que le lait de fabrication fromagère enrichi en phospholipides permettait une augmentation des rendements bruts de la fabrication de certains fromages comme le Cheddar (Mistry et al., 1996, Journal of Dairy Science) et la Mozzarella (Poduval et Mistry, 1999, Journal of Dairy Science).
- De nombreuses études scientifiques ont montré que les protéines laitières, en particulier celles contenues dans le babeurre ont d'excellente qualités nutritionnelles (Léonil et al.,2001, Lait, nutrition et Santé, éditions Technique et Documentation). Contrairement aux protéines végétales, les protéines animales du lait, contiennent tous les acides aminés y compris ceux dits "essentiels" que notre corps ne peut fabriquer et qui doivent impérativement lui être apportés par l'alimentation. Elles ont en plus des caractéristiques spécifiques dont les vertus thérapeutiques commencent à être aujourd'hui bien connues. À titre d'exemple de protéines présentes dans le lait et qui manifestent des activités biologiques intéressantes on peut citer les immunoglobulines, l'α-lactalbumine, la lactoferrine, les lysozymes. À l'heure actuelle, certains médicaments sont développés à partir de protéines extraites du lait de vache, ces médicaments visant notamment à traiter le psoriasis et les maladies inflammatoires de l'intestin, comme alternative aux antibiotiques classiquement utilisés (Université de Laval, Québec).
- Parmi les nombreux bienfaits du calcium, celui le plus connu est son rôle dans la formation et à la croissance des os et donc dans la prévention de l'ostéoporose, mais les études montre que le calcium est aussi bénéfique dans la prévention de troubles aussi divers que l'hypertension artérielle, la lithiase rénale, l'obésité, le cancer colorectal. Le calcium est ainsi utile pour favoriser les régimes ou contrôler le poids, mais également pour la prévention de certains cancers. Selon les experts nutritionnistes, la spéciation chimique du calcium issu du lait et les composants potentiellement favorables à sa biodisponibilité qui lui sont associés en font incontestablement, quantitativement et qualitativement, le calcium alimentaire de référence (L. Guéguen, Cahiers de Nutrition et de Diététique Vol 40, septembre 2005).

La présente invention a également pour objet l'utilisation d'un produit laitier selon l'invention pour la préparation d'un ingrédient alimentaire ou d'une composition alimentaire destiné(e) à agir comme agent anti-athérogènes, agent anti-inflammatoire, agent hypocholestérolémiant, agent anti-infectieux, agent pour la prévention de cancers, de maladies cardio-vasculaires, de maladies auto-immunes, de maladies neurodégénératives, de l'obésité, agent réduisant les troubles liés à la dépression chez les personnes âgées, agent réduisant les troubles liés aux déclins des fonctions cognitives chez les personnes âgées, à l'hypertension artérielle, agent favorisant la résistance à l'effort physique, agent favorisant la détoxification du foie, agent pour la prévention de l'ostéoporose.

On entend par « ingrédient alimentaire » au sens de la présente invention tout ingrédient entrant dans une composition alimentaire.

La présente invention a également pour objet un produit laitier selon l'invention, comme ingrédient alimentaire destiné à agir comme agent anti-athérogènes, agent anti-inflammatoire, agent hypocholestérolémiant, agent anti-infectieux, agent pour la prévention de cancers, de maladies cardio-vasculaires, de maladies auto-immunes, de maladies neurodégénératives, de l'obésité, agent réduisant les troubles liés à la dépression chez les personnes âgées, agent réduisant les troubles liés aux déclins des fonctions cognitives chez les personnes âgées, à l'hypertension artérielle, agent favorisant la résistance à l'effort physique, agent favorisant la détoxification du foie, agent pour la prévention de l'ostéoporose.

La présente invention a également pour objet une composition selon l'invention, ladite composition étant une composition pharmaceutique destinée à la prévention et/ou au traitement d'une pathologie sélectionnée parmi les maladies neurodégénératives, les maladies cardiovasculaires, les maladies infectieuses, les maladies auto-immunes, les cancers, l'ostéoporose, l'obésité et/ou à la prévention et/ou au traitement de troubles sélectionnés parmi les troubles liés aux déclins des fonctions cognitives, les troubles liés à la dépression, les troubles liés à la réponse à inflammatoire, les troubles liés à l'hypertension artérielle.

La présente invention a également pour objet l'utilisation d'un produit laitier selon l'invention en tant qu'agent de texture ou pour la préparation d'un agent de texture.

On entend par « agent de texture » au sens de la présente invention, tout agent agissant sur les propriétés physiques d'un produit telles que la densité, la fluidité, la viscosité, et également sur les sensations gustatives telles que le moelleux, l'onctuosité, le crémeux.

De nombreuses études scientifiques ont montré que les protéines laitières, notamment les caséines, présentent des propriétés d'absorption d'eau, de solubilité, de gélification, d'émulsification, de moussage même après avoir subi des traitements chimiques, physiques ou encore enzymatiques (Cheftel et al., 1982, le lait, 62, 435-483). En outre, certains phospholipides sont couramment utilisés comme agent émulsifiant et comme agent permettant d'améliorer les qualités organoleptiques d'un produit (Alvarez et al., Grasas y Aceites, 2000, 74-96).

Par ailleurs, il a été démontré que l'augmentation de rendement fromager était corrélée aux propriétés texturantes du babeurre. Ainsi, le lait de fabrication fromagère enrichie en phospholipides (ajout de babeurre concentré par ultrafiltration) permet une augmentation des rendements bruts de la fabrication de Cheddar (Mistry et al., 1996, Journal of Dairy Science, 79, 1137-1145) et de Mozzarella (Poduval et Mistry, 1999, Journal of Dairy Science, 82, 1-9). En technologie de fromage fondu, l'addition de quelques pourcentages de babeurre concentré par ultrafiltration permet de diminuer l'exsudation d'huile et du fondant avec une augmentation de la viscosité. À quantité de sels émulsifiants équivalents, la microstructure des fromages fondus est dispersée plus finement que celle des témoins sans babeurre (Raval et Mistry, 1999, Journal of Dairy Science, 82, 2334-2343).

En particulier, l'agent de texture peut être choisi dans le groupe comprenant les agents anti-synérèse, les agents lissant, les agents émulsifiant, les agents d'onctuosité, les agents de texture crémeuse, les agents favorisant le rendement fromager.

Les produits laitiers selon l'invention sous forme déshydratée peuvent être inclus dans des compositions comme agent de texture à une teneur comprise entre 0, 0,5 à 5 %, de préférence entre 0,25 % et 1 % en poids par rapport au poids total de la composition selon la texture recherchée et éventuellement les ferments utilisés.

La présente invention a également pour objet l'utilisation cosmétique d'un produit laitier selon l'invention comme agent émulsifiant et/ou agent hydratant et/ou agent d'encapsulation et/ou agent d'amélioration de la texture de la peau, dans une composition cosmétique.

Les produits laitiers selon l'invention sous forme déshydratée peuvent être inclus dans des compositions cosmétiques à une teneur comprise entre 0,5 et 5 %, de préférence entre 1 et 2,5 %.

La présente invention a également pour objet une composition selon l'invention, ladite composition étant une composition cosmétique destinée à améliorer l'aspect de la peau, la texture de la peau, à hydrater la peau et/ou à prévenir le vieillissement cutané.

La présente invention a également pour objet l'utilisation d'un produit laitier selon l'invention pour la préparation d'une composition cosmétique destinée à améliorer l'aspect de la peau, la texture de la peau, à hydrater la peau et/ou à prévenir le vieillissement cutané.

Les propriétés bénéfiques du produit laitier selon l'invention pour une application cosmétique se définissent notamment au niveau des phospholipides. En effet, il a été montré qu'outre leurs propriétés émulsifiantes, les phospholipides issus du lait ont un effet anti-inflammatoire et un effet hydratant et ainsi améliore la texture, l'aspect et l'hydratation cutanée. En outre les phospholipides issus du lait sont particulièrement intéressants en cosmétique pour leur stabilité, leur résistance à l'oxydation et pour leurs aptitudes à encapsuler des ingrédients cosmétiques particulièrement sensibles, notamment sous formes de liposomes. Il est également bien connu que les céramides jouent un rôle important contre le vieillissement cutané. De plus, les phospholipides sont des composants essentiels pour toutes les membranes biologiques (Alvarez et al., Grasas y Aceites, 2000, 74-96).

Les propriétés bénéfiques du produit laitier selon l'invention pour une application cosmétique se définissent également au niveau des protéines. En effet, il a été montré que les protéines laitières, notamment les caséines, présentent d'excellentes propriétés de texturation mais également d'hydratation (Cheftel et al., 1982, le lait, 62, 435-483).

D'autres avantages et caractéristiques de l'invention apparaîtront au regard des exemples qui suivent. Ces exemples sont donnés à titre indicatif et non limitatif.

### 1. Exemple d'un procédé d'obtention d'un produit laitier selon l'invention

### 1.1 Protocole

200 litres de babeurres obtenu par barattage de la crème de lait de vache, ont été pasteurisés à 60°C pendant 30 minutes dans des cuves Tecnal à double paroi ou à 80°C pendant 1 minute (mode de réalisation préféré à l'échelle industrielle). Au cours du traitement thermique, la matière grasse coalesce et des agrégats apparaissent à la surface du mélange sous agitation.

Le pH du babeurre avant ultrafiltration était soit de 4,6 soit de 5,8 afin d'éviter le colmatage des membranes d'ultrafiltration.

Le babeurre traité thermiquement a ensuite été homogénéisé à 55-60°C (appareil Rannie) à 25 bars ; l'homogénéisation rendant le babeurre parfaitement fluide et homogène.

La concentration par ultrafiltration a été réalisée sur un pilote TIA, équipé de membranes minérales ou organiques avec un seuil de coupure de 10000 Da. La surface totale filtrante était de 2,20 m². Le bac de lancement possédait un volume de 200 litres.

Les conditions d'ultrafiltration en batch (retour du retentât dans le bac de lancement) étaient les suivantes :
- La température du babeurre traité thermiquement et homogénéisé était d'environ 50°C,
- La vitesse de circulation était de 3 m/s,
- la vanne perméat était ouverte (sans contre-pression).

La pression entrée membrane (PE) était de 2 bars et la pression sortie (PS) était de 0 bar. La pression entrée a été réglée progressivement à 5 bars. Le débit moyen du perméat était d'environ 110 1/h/m². Un échangeur d'eau glacée sur la boucle retentât maintenait la température de filtration à environ 50°C. La durée d'ultrafiltration était d'environ 45 minutes. En fin de concentration, la pression d'entrée membrane était de 8.5 bars. Le facteur de réduction volumique (FRV) du babeurre était situé entre 4 et 5.

À la fin d'ultrafiltration, le retentât final fortement visqueux, a été soit déshydraté par atomisation puis conditionné soit directement conditionné en étant pompé, puis stocké à 4°C.

### 1.2 Résultats

Les résultats, ci-après, ont été obtenus à partir de produits issus d'un procédé similaire à celui décrit ci-dessus, au point 1.1.

| | **Babeurre acide** (produit de départ) | **Retentât d'ultrafiltration** | **Poudre du retentât de babeurre** |
|---|---|---|---|
| **Extrait sec** (g/100 g de produit) | 8,3 | 20,0 | 97,5 |
| **pH** | 5,7 | 5,70 | 5,6 |
| **Protéines** (g/100 g de produit) | 2,6 | 12,5 | 60,0 |
| **Protéines sériques** (g/100 g de produit) | 0,36 | 1,0 | 4,5 |
| **Caséines** (g/ 100 g de produit) | 2,24 | 11,5 | 55,5 |
| **Glucides** (g/100 g de produit) | 4,3 | 2,6 | 13,5 |
| **Lipides** (g/100 g de produit) | 0,8 | 4,0 | 20,0 |
| **Phospholipides Totaux** (g/100 g de produit) | 0,1 | 0,60 | 2,6 |
| **Sphingomyéline** (g/100 g de produit) | 0,01 | 0,15 | 0,70 |
| **Cendres** (Minéraux) (g/100 g de produit) | 0,6 | 0,9 | 4,0 |
| **Calcium** (mg/100 g de produit) | 80 | 200 | 950 |
| **Sodium** (mg/100 g de produit) | 30 | 26 | 130 |
| **Potassium** (mg/100 g de produit) | 120 | 90 | 440 |

| **Dosage des phospholipides (mg/100 g de produit) par Chromatographie en Phase Liquide (HPLC) et Détection Diffusion de Lumière (DDL)** | **Babeurre acide** (produit de départ) | **Retentât d'ultrafiltration** | **Poudre de retentât du babeurre** |
|---|---|---|---|
| **Phosphatidyléthanolamine(PE)** | 67 | 280 | 965 |
| **Phosphatidylinositol (PI)** | 2,7 | 16 | 66 |
| **Phosphatidylcholine (PC)** | 26 | 128 | 617 |
| **Phosphatidylsérine (PS)** | 1,5 | 10,5 | 43 |
| **Sphingomyéline (SM)** | 9,8 | 155 | 762 |
| **Acide phosphatidique (PA)** | 1,80 | 26 | 112 |
| **Total des phospholipides** | 108 | 614,5 | 2565 |

Les produits contenant la choline (tableau ci-dessous) ont été dosés par chromatographie liquide couplée avec une spectrométrie de masse à ionisation electrospray et en utilisant la dilution isotopique (dosage simultané de la molécule d'intérêt et son homologue marqué) (LC-ESI-IDMS).

| **Produits** | **Choline libre** | **Phosphatidylcholine** | **Sphingomyléine** | **Phosphocholine** | **Glycerophosphocholine** | **Total choline** |
|---|---|---|---|---|---|---|
| | En mg de choline /100 g de produit | En équivalent mg de choline/100 g de produit | En équivalent mg de choline/100 g de produit | En équivalent mg de choline/100 g de produit | En équivalent mg de choline/100 g de produit | En mg de choline /100 g de produit |
| **Babeurre acide** (produit de départ) | 2,60 | 5,5 | 3,20 | 1,20 | 10,5 | 23 |
| **Retentât d'ultrafiltration** | 2,3 | 30 | 11,5 | 0,64 | 5,95 | 50 |
| **Poudre du retentât de babeurre** | 10.8 | 136 | 52.42 | 2.6 | 22,20 | 224 |

Ainsi, le procédé selon l'invention permet de réduire de moitié la teneur en glucides du babeurre et ainsi d'obtenir un produit présentant environ 10 % de glucides lorsque le babeurre est issu du lait de vache. Parallèlement, le procédé selon l'invention, jusqu'à l'étape c) incluse, permet de concentrer environ 5 fois la quantité de protéines présentes dans le babeurre, environ 2,5 fois la quantité de calcium présent dans le babeurre, environ 6 fois la quantité de phospholipides totaux dont 15 fois la quantité de sphingomyéline et 2 fois la quantité de choline présents dans le babeurre.

En outre, le procédé selon l'invention incluant l'étape d) de déshydratation permet de concentrer environ 20 fois la quantité de protéines présentes dans le babeurre, environ 10 fois la quantité de calcium présent dans le babeurre, environ 25 fois la quantité de phospholipides totaux dont 70 fois la quantité de sphingomyéline et environ 10 fois la quantité de choline présents dans le babeurre.

### II. Exemple de compositions alimentaires comprenant un produit laitier selon l'invention

Les produits laitiers selon l'invention utilisés ci-après ont été obtenus selon un procédé similaire à celui décrit dans l'exemple 1. Dans les compositions alimentaires décrites, ci-après, les produits laitiers selon l'invention ne sont pas déshydratés et correspondent aux produits obtenus à l'étape c) du procédé selon l'invention.

### II.1 Formulations de compositions alimentaires comprenant un produit laitier selon l'invention

| **Matières Premières (%)** | **Dessert lacté Tatin** | **Dessert lacté vanille** |
|---|---|---|
| Saccharose | 7 à 8 | 8 à 9 |
| Huile de colza | 1 à 1,3 | 1 à 1,3 |
| Lécithine de soja | 0,05 à 0,08 | 0,05 à 0,08 |
| Lait écrémé poudre | 0,8 à 1 | 1 à 1,3 |
| Amidon natif de riz | 1 à 2 | 1 à 2 |
| Extrait d'algue - Carraghenane | 0,2 à 0,4 | 0,2 à 0,4 |
| Gomme Xanthane | 0,04 à 0,06 | 0,04 à 0,06 |
| Fibre soluble d'acacia | 1 à 1,7 | 1 à 1,7 |
| Caramel aromatique | 1 à 1,7 | |
| Arômes Tatin | 0,05 à 0,08 | |
| Garniture Fruit - Pomme Tatin | 12 à 16 | |
| Arômes Vanille | | 1,2 à 1,5 |
| Colorant Bêta Carotène | | 0,01 à 0,015 |
| **Retentât d'ultrafiltration (Produit laitier selon l'invention)** | 40 à 48 | 48 à 52 |
| Eau | QSP pour 100 | QSP pour 100 |
| TOTAL | 100 | 100 |

| **Matières Premières (%)** | **Boisson fruits rouges - Litchi** |
|---|---|
| Pectine | 0,2 à 0,5 |
| Saccharose | 8 à 10 |
| Lécithine de soja | 0,05 à 0,08 |
| Amidon natif de riz | 0,4 à 0,7 |
| Fibre d'acacia | 1 à 1,5 |
| Arôme Litchi - Fruits rouges | 0,5 à 0,8 |
| Concentré d'airelle (« Cranberry ») | 1 à 2 |
| **Retentât d'ultrafiltration (Produit laitier selon l'invention)** | 50 à 55 |
| Eau | QSP 100 |
| TOTAL | 100 |

### II.2 Résultats d'analyses biochimiques de compositions alimentaires comprenant un produit laitier selon l'invention

Les compositions alimentaires décrites au point II.1, ci-dessus ont été analysées :

| | **Dessert lacté Tatin** | **Dessert lacté vanille** | **Boisson fruits rouges** - **Litchi** |
|---|---|---|---|
| **Protéines (%)** | 4,20 | 4,40 | 6,20 |
| **Lipides (%)** | 2,0 | 2,30 | 1,70 |
| **Glucides (%)** | 13,8 | 13,50 | 12,20 |
| **Calcium** (mg/100 g de produit) | 100 | 100 | 95 |
| - **Phospholipides** | 310 | 310 | 310 |
| - **Choline** (mg/100 g de produit) | 25 | 25 | 25 |
| **Cholestérol** (mg/100 g de produit) | 6 | 6 | 6 |
| **Valeur énergétique** (Kcal/100 g de produit) | 90 | 92 | 90 |

En 2001, l'Administration Américaine des Denrées alimentaires et Médicaments (« Food and Drug Administration », FDA) a autorisé des allégations nutritionnelles sur des produits contenant de la choline. Ainsi, les aliments contenant plus de 110 mg de choline par portion peuvent déclarer être une « excellente source de choline ». Les aliments contenant plus de 55 mg peuvent déclarer être une « bonne source de choline ».

Une portion de 250 ml de boisson formulée (par exemple, une boisson fruits rouges - Litchi telle que décrite ci-dessus) comprenant 50 à 55 % de retentât d'ultrafiltration (produit laitier selon l'invention) apporte environ 62 mg de choline et peut donc être considérée comme une bonne source de choline.

De même une portion de 250 g de dessert lacté (par exemple, un dessert lacté Tatin ou vanille tel que décrit ci-dessus) comprenant de 40 à 50 % de retentât d'ultrafiltration (produit laitier selon l'invention) apporte environ 62 mg de choline et peut donc être considérée comme une bonne source de choline.

## Revendications

1. Procédé de préparation d'un produit laitier, **caractérisé en ce qu'**il comprend les étapes suivantes :
a) traitement thermique d'un babeurre à une température comprise entre 50 et 90 °C, de préférence entre 60 et 80 °C et tout préférentiellement à 80 °C, pendant une durée comprise entre 30 secondes et 60 minutes de préférence entre 1 et 30 minutes, et tout préférentiellement 1 minute ;
b) homogénéisation dudit babeurre traité thermiquement obtenu à l'étape a)
c) ultrafiltration du babeurre traité thermiquement et homogénéisé obtenu à l'étape b) à un pH compris dans une gamme allant de 4,5 à 5,8 ou de 5,5 à 6, de préférence à pH 4,7 ou pH 5,8 et tout préférentiellement à pH 5,8, sur des membranes ayant un seuil de coupure compris entre 1 000 et 20 000 Da, de préférence entre 5 000 et 15 000 Da, et tout préférentiellement de 10 000 Da.

2. Procédé selon la revendication 1, **caractérisé en ce que** l'étape b) d'homogénéisation est mise en oeuvre à une température comprise entre 40 et 60 °C, de préférence entre 45 et 55 °C, et tout préférentiellement à 50 °C et à une pression comprise entre 160 et 260 bars, de préférence entre 180 et 240 bars, et tout préférentiellement à 220 bars.

3. Procédé selon l'une des revendications 1 ou 2, **caractérisé en ce que** l'étape c) d'ultrafiltration est mise en oeuvre sur des membranes organiques, en particulier sur des membranes organiques spiralées.

4. Procédé selon l'une quelconque des revendications 1 à 3, **caractérisé en ce qu'**au moins une des étapes a) et b) est mise en oeuvre à un pH compris dans une gamme allant de 4,5 à 5,8 ou de 5,5 à 6, de préférence à pH 4,7 ou pH 5,8 et tout préférentiellement à pH 5,8.

5. Procédé selon l'une quelconque des revendications 1 à 4, **caractérisé en ce qu'**il comprend en outre, l'étape suivante :
d) déshydratation du retentât obtenu à l'étape c), de préférence par atomisation.

6. Procédé selon l'une quelconque des revendications 1 à 5, **caractérisé en ce qu'**il comprend en outre l'étape suivante :
e) conditionnement du retentât obtenu à l'étape c) ou du retentât déshydraté obtenu à l'étape d).

7. Composition comprenant un produit laitier obtenu grâce au procédé selon l'une quelconque des revendications 1 à 6, ladite composition étant choisie dans le groupe comprenant les compositions alimentaires, les compositions pharmaceutiques, et les compositions cosmétiques.

8. Utilisation non thérapeutique d'un produit laitier obtenu grâce au procédé selon l'une quelconque des revendications 1 à 6, ou d'une composition selon la revendication 7, ladite composition étant une composition alimentaire, en vue d'un apport en protéines, calcium et/ou phospholipides, en particulier en choline et/ou sphingomyéline.

9. Utilisation d'un produit laitier obtenu grâce au procédé selon l'une quelconque des revendications 1 à 6, pour la préparation d'un ingrédient alimentaire ou d'une composition alimentaire destiné(e) à agir comme agent anti-athérogènes, agent anti-inflammatoire, agent hypocholestérolémiant, agent anti-infectieux, agent pour la prévention de cancers, de maladies cardio-vasculaires, de maladies auto-immunes, de maladies neurodégénératives, de l'obésité, agent réduisant les troubles liés à la dépression chez les personnes âgées, agent réduisant les troubles liés aux déclins des fonctions cognitives chez les personnes âgées, agent réduisant les troubles liés à l'hypertension artérielle, agent favorisant la résistance à l'effort physique, agent favorisant la détoxification du foie, agent pour la prévention de l'ostéoporose.

10. Produit laitier obtenu grâce au procédé selon l'une quelconque des revendications 1 à 6, comme ingrédient alimentaire destiné à agir comme agent anti-athérogènes, agent anti-inflammatoire, agent hypocholestérolémiant, agent anti-infectieux, agent pour la prévention de cancers, de maladies cardio-vasculaires, de maladies auto-immunes, de maladies neurodégénératives, l'obésité, agent réduisant les troubles liés à la dépression chez les personnes âgées, à l'hypertension artérielle, agent réduisant les troubles liés aux déclins des fonctions cognitives chez les personnes âgées, agent favorisant la résistance à l'effort physique, agent favorisant la détoxification du foie, agent pour la prévention de l'ostéoporose.

11. Composition selon la revendication 7, **caractérisée en ce que** la composition est une composition pharmaceutique destinée à la prévention et/ou au traitement d'une pathologie sélectionnée parmi les maladies neurodégénératives, les maladies cardiovasculaires, les maladies infectieuses, les maladies auto-immunes, les cancers, l'ostéoporose, l'obésité et/ou à la prévention et/ou au traitement de troubles sélectionnés parmi les troubles liés aux déclins des fonctions cognitives, les troubles liés à la dépression, les troubles liés à la réponse à inflammatoire, les troubles liés à l'hypertension artérielle.

12. Utilisation d'un produit laitier obtenu grâce au procédé selon l'une quelconque des revendications 1 à 6, en tant qu'agent de texture ou pour la préparation d'un agent de texture.

13. Utilisation selon la revendication 12, **caractérisé en ce que** l'agent de texture est choisi dans le groupe comprenant les agents anti-synérèse, les agents lissant, les agents émulsifiant, les agents d'onctuosité, les agents de texture crémeuse, les agents favorisant le rendement fromager.

14. Utilisation cosmétique d'un produit laitier obtenu grâce au procédé selon l'une quelconque des revendications 1 à 6, comme agent émulsifiant et/ou agent hydratant et/ou agent d'encapsulation et/ou agent d'amélioration de la texture de la peau, dans une composition cosmétique.

15. Composition selon la revendication 7, **caractérisée en ce que** la composition est une composition cosmétique destinée à améliorer l'aspect de la peau, la texture de la peau, à hydrater la peau et/ou à prévenir le vieillissement cutané.

16. Utilisation d'un produit laitier obtenu grâce au procédé selon l'une quelconque des revendications 1 à 6, pour la préparation d'une composition cosmétique destinée à améliorer l'aspect de la peau, la texture de la peau, à hydrater la peau et/ou à prévenir le vieillissement cutané.

## Claims

1. Method for preparing a dairy product, **characterised in that** it comprises the following steps:
a) heat-treating a buttermilk at a temperature between 50 and 90°C, preferably between 60 and 80°C and more preferentially at 80°C, for a time between 30 seconds and 60 minutes, preferably between 1 and 30 minutes, and more preferentially 1 minute;
b) homogenising said heat-treated buttermilk obtained in step a)
c) ultrafiltration of the homogenised heat-treated buttermilk obtained in step b) at a pH within a range from 4.5 to 5.8 or from 5.5 to 6, preferably at pH 4.7 or pH 5.8 and more preferentially at pH 5.8, on membranes with a cut-off threshold between 1000 and 20,000 Da, preferably between 5000 and 15,000 Da and more preferentially of 10,000 Da.

2. Method according to claim 1, **characterised in that** the homogenisation step b) is implemented at a temperature between 40 and 60°C, preferably between 45 and 55°C and more preferentially at 50°C and at a pressure between 160 and 260 bar, preferably between 180 and 240 bar, and more preferentially at 220 bar.

3. Method according to any of claims 1 or 2, **characterised in that** the ultrafiltration step c) is implemented on organic membranes, particularly on spiral-wound organic membranes.

4. Method according to any of claims 1 to 3, **characterised in that** at least one of steps a) and b) is implemented at a pH within a range from 4.5 to 5.8 or from 5.5 to 6, preferably at pH 4.7 or pH 5.8 and more preferentially at pH 5.8.

5. Method according to any of claims 1 to 4, **characterised in that** it further comprises the following step:
d) drying the retentate obtained in step c), preferably by spray drying.

6. Method according to any of claims 1 to 5, **characterised in that** it further comprises the following step:
e) packaging the retentate obtained in step c) or the dried retentate obtained in step d).

7. Composition comprising a dairy product obtained using the method according to any of claims 1 to 6, said composition being chosen from the group comprising nutritional compositions, pharmaceutical compositions and cosmetic compositions.

8. Non-therapeutic use of a dairy product obtained using the method according to any of claims 1 to 6, or of a composition according to claim 7, wherein said composition is a nutritional composition, with a view to providing protein, calcium and/or phospholipids, particularly choline and/or sphingomyelin.

9. Use of a dairy product obtained using the method according to any of claims 1 to 6, for preparing a nutritional ingredient or a nutritional composition intended to act as an antiatherogenic agent, anti-inflammatory agent, hypocholesterolaemic agent, anti-infective agent, agent for preventing cancer, cardiovascular diseases, autoimmune diseases, neurodegenerative diseases, obesity, agent reducing depression- or arterial hypertension-related disorders in the elderly, agent reducing cognitive function decline-related disorders in the elderly, agent promoting physical stamina, agent promoting liver detoxification, agent for preventing osteoporosis.

10. Dairy product obtained using the method according to any of claims 1 to 6, as a nutritional ingredient intended to act as an antiatherogenic agent, anti-inflammatory agent, hypocholesterolaemic agent, anti-infective agent, agent for preventing cancer, cardiovascular diseases, autoimmune diseases, neurodegenerative diseases, obesity, agent reducing depression-related disorders in the elderly, agent reducing cognitive function decline-related disorders in the elderly, agent reducing arterial hypertension-related disorders, agent promoting physical stamina, agent promoting liver detoxification, agent for preventing osteoporosis.

11. Composition according to claim 7, **characterised in that** the composition is a pharmaceutical composition for preventing and/or treating a condition selected from neurodegenerative diseases, cardiovascular diseases, infectious diseases, autoimmune diseases, cancer, osteoporosis, obesity and/or preventing and/or treating disorders selected from cognitive function decline-related disorders, depression-related disorders, inflammatory response-related disorders, arterial hypertension-related disorders.

12. Use of a dairy product obtained using the method according to any of claims 1 to 6, as a texture agent or for preparing a texture agent.

13. Use according to claim 12, **characterised in** the texture agent is chosen from the group comprising anti-syneresis agents, smoothing agents, emulsifying agents, oiliness agents, creamy texture agents, agents promoting cheese yield.

14. Cosmetic use of a dairy product obtained using the method according to any of claims 1 to 6, as an emulsifying agent and/or moisturising agent and/or encapsulation agent and/or skin texture enhancement agent, in a cosmetic composition.

15. Composition according to claim 7, **characterised in that** the composition is a cosmetic composition for enhancing skin appearance, skin texture, moisturising the skin and/or preventing skin ageing.

16. Use of a dairy product obtained using the method according to any of claims 1 to 6, for preparing a cosmetic composition for enhancing skin appearance, skin texture, moisturising the skin and/or preventing skin ageing.

## Patentansprüche

1. Verfahren zur Herstellung eines Milchprodukts, **dadurch gekennzeichnet, dass** es die folgenden Schritte umfasst:
a) Wärmebehandlung einer Buttermilch bei einer Temperatur zwischen 50 und 90°C, vorzugsweise zwischen 60 und 80°C und sehr vorzugsweise bei 80°C während einer Dauer zwischen 30 Sekunden und 60 Minuten, vorzugsweise zwischen 1 und 30 Minuten und sehr vorzugsweise während 1 Minute;
b) Homogenisierung der in Schritt a) erhaltenen wärmebehandelten Buttermilch;
c) Ultrafiltration der in Schritt b) erhaltenen wärmebehandelten und homogenisierten Buttermilch bei einem pH-Wert im Bereich von 4,5 bis 5,8 oder von 5,5 bis 6, vorzugsweise bei einem pH-Wert von 4,7 oder 5,8 und sehr vorzugsweise bei einem pH-Wert von 5,8 auf Membranen mit einer Trennschwelle zwischen 1.000 und 20.000 Da, vorzugsweise zwischen 5.000 und 15.000 Da und sehr vorzugsweise von 10.000 Da.

2. Verfahren nach Anspruch 1, **dadurch gekennzeichnet, dass** der Schritt b) zur Homogenisierung bei einer Temperatur zwischen 40 und 60°C, vorzugsweise zwischen 45 und 55°C und sehr vorzugsweise bei 50°C und bei einem Druck zwischen 160 und 260 bar, vorzugsweise zwischen 180 und 240 bar und sehr vorzugsweise bei 220 bar durchgeführt wird.

3. Verfahren nach einem der Ansprüche 1 oder 2, **dadurch gekennzeichnet, dass** der Schritt c) zur Ultrafiltration auf organischen Membranen, insbesondere auf organischen spiralgewickelten Membranen durchgeführt wird.

4. Verfahren nach einem beliebigen der Ansprüche 1 bis 3, **dadurch gekennzeichnet, dass** mindestens einer der Schritte a) und b) bei einem pH-Wert in einem Bereich von 4,5 bis 5,8 oder von 5,5 bis 6, vorzugsweise bei einem pH-Wert von 4,7 oder 5,8 und sehr vorzugsweise bei einem pH-Wert von 5,8 durchgeführt wird.

5. Verfahren nach einem beliebigen der Ansprüche 1 bis 4, **dadurch gekennzeichnet, dass** es darüber hinaus den folgenden Schritt umfasst:
d) Dehydratation des in Schritt c) erhaltenen Retentats, vorzugsweise durch Atomisierung.

6. Verfahren nach einem beliebigen der Ansprüche 1 bis 5, **dadurch gekennzeichnet, dass** es darüber hinaus den folgenden Schritt umfasst:
e) Aufbereitung des in Schritt c) erhaltenen Retentats oder des in Schritt d) erhaltenen dehydratisierten Retentats.

7. Zusammensetzung umfassend ein mithilfe des Verfahrens nach einem der Ansprüche 1 bis 6 erhaltenes Milchprodukt, wobei diese Zusammensetzung aus der Gruppe gewählt wird, die Nahrungsmittelzusammensetzungen, pharmazeutische Zusammensetzungen und kosmetische Zusammensetzung umfasst.

8. Nichttherapeutische Verwendung eines mithilfe des Verfahrens nach einem der Ansprüche 1 bis 6 erhaltenen Milchprodukts oder einer Zusammensetzung nach Anspruch 7, wobei diese Zusammensetzung eine Nahrungsmittelzusammensetzung ist, zur Verabreichung von Proteinen, Calcium und/oder Phospholipiden, insbesondere von Cholin und/oder Sphingomyelin.

9. Verwendung eines mithilfe des Verfahrens nach einem beliebigen der Ansprüche 1 bis 6 erhaltenen Milchprodukts zur Zubereitung eines Nahrungsmittelbestandteils oder einer Nahrungsmittelzusammensetzung, die dazu dient, als antiatherogenes Mittel, entzündungshemmendes Mittel, cholesterinsenkendes Mittel, Antiinfektionsmittel, Mittel zur Vorbeugung von Krebs, Herz-Kreislauf-Erkrankungen, Autoimmun-Erkrankungen, neurodegenerativen Erkrankungen Fettleibigkeit, depressionsbedingte Störungen bei älteren Menschen verringerndes Mittel, mit dem Abbau der kognitiven Fähigkeiten verbundene Störungen bei älteren Menschen verringerndes Mittel, mit Bluthochdruck verbundene Störungen verringerndes Mittel, die körperliche Belastbarkeit förderndes Mittel, die Entgiftung der Leber förderndes Mittel, Mittel zur Vorbeugung von Osteoporose zu wirken.

10. Milchprodukt, das mithilfe des Verfahrens nach einem beliebigen der Ansprüche 1 bis 6 erhaltenen wurde, als Nahrungsmittelbestandteil, der dazu dient, als antiatherogenes Mittel, entzündungshemmendes Mittel, cholesterinsenkendes Mittel, Antiinfektionsmittel, Mittel zur Vorbeugung von Krebs, Herz-Kreislauf-Erkrankungen, Autoimmun-Erkrankungen, neurodegenerativen Erkrankungen Fettleibigkeit, depressionsbedingte Störungen bei älteren Menschen verringerndes Mittel, mit Bluthochdruck verbundene Störungen, mit dem Abbau der kognitiven Fähigkeiten verbundene Störungen bei älteren Menschen verringerndes Mittel, die körperliche Belastbarkeit förderndes Mittel, die Entgiftung der Leber förderndes Mittel, Mittel zur Vorbeugung von Osteoporose zu wirken.

11. Zusammensetzung nach Anspruch 7, **dadurch gekennzeichnet, dass** die Zusammensetzung eine pharmazeutische Zusammensetzung ist, die bestimmt ist zur Vorbeugung und/oder Behandlung einer Krankheit ausgewählt unter den neurodegenerativen Erkrankungen, den Herz-Kreislauf-Erkrankungen, den Infektionskrankheiten, den Autoimmun-Erkrankungen, Krebs, Osteoporose, Fettleibigkeit und/oder zur Vorbeugung und/oder Behandlung von Störungen ausgewählt unter den mit dem Abbau der kognitiven Fähigkeiten verbundenen Störungen, den depressionsbedingten Störungen, den mit der Entzündungsantwort verbundenen Störungen und den mit Bluthochdruck verbundenen Störungen.

12. Verwendung eines mithilfe des Verfahrens nach einem beliebigen der Ansprüche 1 bis 6 erhaltenen Milchprodukts als Texturmittel oder zur Zubereitung eines Texturmittels.

13. Verwendung nach Anspruch 12, **dadurch gekennzeichnet, dass** das Texturmittel aus der Gruppe gewählt wird, die Synäresehemmer, Glättungsmittel, Emulgatoren, Cremigkeit bewirkende Mittel, eine sahnige Textur bewirkende Mittel und Mittel zur Förderung des Käseertrags umfasst.

14. Kosmetische Verwendung eines mithilfe des Verfahrens nach einem beliebigen der Ansprüche 1 bis 6 erhaltenen Milchprodukts als Emulgator und/oder Befeuchtungsmittel und/oder Verkapselungsmittel und/oder Mittel zur Verbesserung der Hauttextur in einer kosmetischen Zusammensetzung.

15. Zusammensetzung nach Anspruch 7, **dadurch gekennzeichnet, dass** die Zusammensetzung eine kosmetische Zusammensetzung ist, die dazu dient, das Aussehen der Haut und die Textur der Haut zu verbessern, die Haut zu befeuchten und/oder der Hautalterung vorzubeugen.

16. Verwendung eines mithilfe des Verfahrens nach einem beliebigen der Ansprüche 1 bis 6 erhaltenen Milchprodukts zur Zubereitung einer kosmetischen Zusammensetzung, die dazu dient, das Aussehen der Haut und die Textur der Haut zu verbessern, die Haut zu befeuchten und/oder der Hautalterung vorzubeugen.
